Europäisches Patentamt

European Patent Office

Office européen des brevets

(19)

(11) Publication number: **0 508 631 A1**

# EUROPEAN PATENT APPLICATION

(12)

(21) Application number: 92302425.1

(22) Date of filing: 20.03.92

(51) Int. Cl.5: **C07C 17/00**, C07C 17/38, C07C 19/08

(30) Priority: 08.04.91 GB 9107340
08.07.91 GB 9114686

(43) Date of publication of application:
14.10.92 Bulletin 92/42

(84) Designated Contracting States:
AT BE CH DE DK ES FR GB GR IT LI LU NL SE

(71) Applicant: IMPERIAL CHEMICAL INDUSTRIES PLC
Imperial Chemical House, Millbank
London SW1P 3JF(GB)

(72) Inventor: Clayton, Peter Paul
5 Chatterton Drive
Lower Norton, Runcorn(GB)

(74) Representative: Oldroyd, Alan et al
ICI Group Patents Services Dept. PO Box 6
Shire Park Bessemer Road
Welwyn Garden City Herts, AL7 1HD(GB)

(54) Production of hydrofluorocarbons.

(57) A process for the production of a hydrofluorocarbon which process comprises contacting a fluorocarbon containing at least one atom of chlorine, bromine or iodine with a complex metal hydride and optionally a metal hydride.

The fluorocarbon containing at least one atom of chlorine, bromine or iodine may be an impurity in a mixture thereof with a hydrofluoroalkane.

This invention relates to the production of hydrofluorocarbons, and in particular hydrofluoroalkanes, from fluorocarbons containing at least one atom of chlorine, bromine or iodine by a reduction process in which the atom(s) of chlorine, bromine or iodine is/are replaced by hydrogen. The process is also useful for the removal of chlorine containing impurities, in particular highly toxic chlorine-containing impurities, from hydrofluoroalkanes.

In recent years chlorofluorocarbons (CFCs), which are used on a large scale around the world, have been perceived as having an adverse effect on the ozone layer and/or as contributing to global warming. Chlorofluorocarbons are used, for example, as refrigerants, as foam blowing agents, as cleaning solvents and as propellants for aerosol sprays in which the variety of applications is virtually unlimited. Consequently, much effort is being devoted to finding suitable replacements for chlorofluorocarbons which will perform satisfactorily in the many applications in which chlorofluorocarbons are used but which will not have the aforementioned environmentally harmful effects. One approach in the search for suitable replacements has centred on fluorocarbons which do not contain chlorine but which may contain hydrogen. For example, the hydrofluoroalkanes, 1,1,1,2-tetrafluoroethane, also known as HFA 134a, difluoromethane, also known as HFA 32, and pentafluoroethane, also known as HFA 125 are of interest as such replacements.

A number of processes have been proposed for the production of hydrofluorocarbons from chloro-(hydro)fluorocarbons, including for example (a) reaction of the chloro(hydro)fluorocarbon with hydrogen fluoride in the vapour phase over a catalyst such as chromia, a halogenated chromia or chromium oxyhalide and (b) hydrogenation of the chloro(hydro)fluorocarbon with hydrogen gas in the vapour phase over a hydrogenation catalyst, usually under superatmospheric pressure.

Such processes suffer from the disadvantages of requiring elevated temperatures and expensive plant and they also tend to lack selectivity with consequent production of numerous unwanted by-products which in turn requires expensive purification procedures.

We have now found a simple process for the production of hydrofluorocarbons from fluorocarbons containing at least one atom of chlorine, bromine or iodine with a high degree of selectivity and in high yields.

According to the present invention there is provided a process for the production of a hydrofluorocarbon which process comprises contacting a fluorocarbon containing at least one atom of chlorine, bromine or iodine with a complex metal hydride.

In the process of the invention, contact of the fluorocarbon containing at least one atom of chlorine, bromine or iodine (hereafter referred to as the starting fluorocarbon) with the complex metal hydride may result in highly selective replacement of the chlorine, bromine or iodine atoms. Indeed we have found that with certain preferred complex metal hydrides, the selectivity of the process may be as high as 95% and even higher; selectivities approaching 100% are usually achieved.

The starting fluorocarbon will usually contain at least one atom of chlorine, since chlorofluorocarbons are the most readily available of the halocarbon starting materials. In this case the process results in replacement of the chlorine atom(s) by hydrogen. For simplicity, the invention will be described hereafter with reference to starting fluorocarbons which contain chlorine, although it is to be understood that the invention is not limited to such starting materials.

The starting fluorocarbon may contain 1 to about 6 carbon atoms, although usually the starting fluorocarbon will be a single carbon or two carbon species. The starting fluorocarbon may be saturated or unsaturated, although it is usually a saturated species, since the desired hydrofluoroalkanes are themselves saturated species.

The starting fluorocarbon may contain only one chlorine atom or it may contain more than one chlorine atom, for example two chlorine atoms. Furthermore, the starting fluorocarbon may contain atoms other than carbon and halogen, for example the starting fluorocarbon may also contain hydrogen atoms. Indeed, the selectivity of the process is highest where the starting fluorocarbon contains at least two fluorine atoms for each carbon atom present.

Preferred starting materials are saturated fluorocarbons comprising one or two carbon atoms and one or two chlorine atoms, for example chlorodifluoromethane, dichlorodifluoromethane, chlorotetrafluoroethane, dichlorotetrafluoroethane and chloropentafluoroethane.

The process of the invention is particularly useful for the production of difluoromethane, tetrafluoroethane or pentafluoroethane.

According to a first preferred embodiment of the invention there is provided a process for the production of difluoromethane which process comprises contacting chlorodifluoromethane or dichlorodifluoromethane with a complex metal hydride.

According to a second preferred embodiment of the invention there is provided a process for the production of tetrafluoroethane (particularly 1,1,1,2-tetrafluoroethane) which process comprises contacting chlorotetrafluoroethane or dichlorotetrafluoroethane (particularly 1,1,1,2-tetrafluorochloroethane or 1,1,1,2-tetrafluorodichloroethane) with a complex metal hydride.

According to a third preferred embodiment of the invention there is provided a process for the production of pentafluoroethane which process comprises contacting chloropentafluoroethane with a complex metal hydride.

The starting fluorocarbons are generally volatile compounds with boiling points below 0°C and the process is often conveniently carried out by contacting the starting fluorocarbon in the vapour phase with the complex metal hydride at room temperature. For example, the starting fluorocarbon may be passed through a solution of the complex metal hydride. The contact time between the starting fluorocarbon and the complex metal hydride may be varied within a wide range without significantly affecting the selectivity of the process. The reaction is essentially instantaneous and high conversions and good selectivities may be achieved even when the contact time is as low as 0.1 seconds even at room temperature; the contact time is usually no more than 1 or 2 seconds.

The temperature and pressure at which the process is carried out are dependent at least to some extent on the particular starting fluorocarbon and the complex metal hydride used. The temperature and pressure may be varied within wide ranges but preferably are such that the starting fluorocarbon and product hydrofluorocarbon are in the vapour phase and the complex metal hydride is in the form of a solution. The temperature will typically be in the range -80°C to 160°C but preferably will be at least -20°C, more preferably at least -10°C and most preferably at least 0°C, and be not more than 60°C, more preferably not more than 40°C and most preferably not more than 20°C. The pressure is preferably ambient pressure.

Complex metal hydrides are well known materials, and any of the known complex metal hydrides may be used. The complex metal hydride is preferably a hydride of a complex of a Group 1A metal, for example lithium or sodium, and a Group 3 metal, for example aluminium or boron; in particular lithium aluminium hydride ($LiAlH_4$) and sodium borohydride ($NaBH_4$). $LiAlH_4$ is preferred. In addition to these hydrides, complex metal hydrides in which one or more of the hydrogen atoms have been replaced by alkoxy groups may also be used, for example lithium triethoxyaluminium hydride $LiAlH(OCH_2CH_3)_3$, lithium tritertiarybutoxyaluminium hydride $LiAlH(OC(CH_3)_3)_3$ and sodium bis(2-methoxyethoxy)aluminium hydride $NaAlH_2$-$(OCH_2CH_2OCH_3)_2$.

The complex metal hydride is preferably provided as a solution through which the starting fluorocarbon is passed. The solvent is preferably a solvent with which the complex metal hydride does not react. The solvent chosen will therefore depend upon the particular complex metal hydride to be used. In the case where a complex metal hydride which is a strong reducing agent is used, for example $LiAlH_4$, the solvent is preferably aprotic, for example tetrahydrofuran, 1,2-dimethoxyethane, diglyme or diethyl ether. However, where the complex metal hydride is a weaker reducing agent, for example $NaBH_4$, the solvent may, in addition to aprotic solvents, be a protic solvent, for example methanol, ethanol or isopropanol.

The concentration of the complex metal hydride in the solvent may be varied within a wide range although typically the solution may be from 0.5M to 5M in the complex metal hydride, for example $LiAlH_4$.

In a further preferred embodiment of the invention, the process is carried out in the presence of a complex metal hydride and a metal hydride, for example an alkali metal hydride since the metal hydride acts to regenerate the complex metal hydride, the complex metal hydride then acting as a hydrogen carrier, thereby prolonging the activity of the complex metal hydride; i.e. where metal hydride is added, the complex metal hydride adopts a catalytic type activity. Furthermore, the addition of the metal hydride also improves the yield of hydrofluorocarbon product from the process. A particularly preferred metal hydride is lithium hydride and a preferred combination of complex metal hydride and metal hydride is lithium aluminium hydride and lithium hydride.

The metal hydride is preferably added in as large a quantity as possible, and where the complex metal hydride is provided as a solution, the solution may be saturated with the metal hydride. Indeed, sufficient metal hydride is preferably added to the solution of complex metal hydride so as to produce a solution saturated with metal hydride and in which solid metal hydride is also present so that as the metal hydride is consumed during the course of the process, solid metal hydride dissolves to maintain the solution saturated with metal hydride.

The proportion of metal hydride to complex metal hydride is dependent upon the particular metal hydride/complex metal hydride combination employed. Usually however, the metal hydride will be present in at least a 2:1 molar excess over the complex metal hydride, and where the metal hydride/complex metal hydride combination is lithium hydride/lithium aluminium hydride, the lithium hydride is preferably present in at least a 5:1 molar excess, especially a 10:1 molar excess over the lithium aluminium hydride.

In this preferred embodiment of the process where a metal hydride is also present, the process may be carried out at higher temperatures than where no metal hydride is present. The temperature may be for example, greater than 30°C, preferably greater than 50°C and more preferably greater than 60°C.

As a result of the excellent conversions and selectivities which may be achieved by the process of the invention, little purification of the product may be required. However, small amounts of unreacted starting fluorocarbon and possibly solvent vapour may be present in the product although by appropriate choice of the process conditions as described previously the amounts of unreacted starting fluorocarbon and solvent present in the product may be very small. These impurities may be removed by any of the known methods for removing such impurities from hydrofluorocarbons, for example, the solvent may be removed by distillation or absorption into water and the unreacted fluorocarbon may be separated by adsorption over a molecular sieve. However, any of the known methods may be used for the removal of such impurities.

The extremely high selectivities with which chlorine is replaced by hydrogen, in molecules also containing fluorine, by the process of the invention may also be exploited in processes for the removal of chlorinated fluorocarbon impurities from hydrofluoroalkanes, for example HFA 134a, HFA 125 and HFA 32. The impurities are removed by conversion into molecules not containing chlorine. In this case, the fluorocarbon containing at least one atom of chlorine which is contacted with the complex metal hydride in the process of the invention is an impurity in a mixture thereof with a hydrofluoroalkane.

Thus, according to a further aspect of the invention there is provided a process for the removal of fluorocarbons containing at least one atom of chlorine, bromine or iodine from mixtures thereof with hydrofluoroalkanes which process comprises contacting the mixture with a complex metal hydride. Preferably a metal hydride is also present.

The hydrofluoroalkane from which the chlorine-containing fluorocarbon impurity is removed by the process of this further aspect of the invention comprises only hydrogen fluorine and carbon and typically comprises 1 or 2 carbon atoms although it may comprise more, say up to 6 carbon atoms. The hydrofluoroalkane may be, for example 1,1,1,2-tetrafluoroethane (HFA 134a), difluoromethane (HFA 32) or pentafluoroethane (HFA 125).

The chlorine-containing fluorocarbon impurity which is removed by the process of this further aspect of the invention will typically comprise at least one atom of chlorine, that is, it will be a chlorofluorocarbon or hydrochlorofluorocarbon.

The process of this further aspect of the invention is particularly useful for removing, by converting to a less toxic non-chlorinated molecule, highly toxic impurities such as may be formed, for example, as by-products in many processes which have been proposed for the production of hydrofluoroalkanes. Thus, for example, the mixture which is treated may be the product mixture obtained from a process for the production of 1,1,1,2-tetrafluoroethane which comprises the very toxic impurity 1-chloro-2,2-difluoroethylene (HCFC 1122), or the product mixture obtained from a process for the production of difluoromethane which also comprises the extremely toxic impurity chlorofluoromethane (HCFC 31).

According to a first preferred embodiment of this further aspect of the invention there is provided a process for the removal of 1-chloro-2,2-difluoroethylene from a mixture thereof with 1,1,1,2-tetrafluoroethane which process comprises contacting the mixture with a complex metal hydride.

According to a second preferred embodiment there is provided a process for the removal of chlorofluoromethane from a mixture thereof with difluoromethane which process comprises contacting the mixture with a complex metal hydride.

Additionally the mixture treated by this further aspect of the invention may be a mixture resulting from a "retrofitting procedure". Thus, whilst the provision of hydrofluoroalkanes in new equipment in which they are to be employed may be straightforward, particular problems arise in relation to existing equipment which contains CFCs and/or HCFCs but which need to be "retrofitted" with the HFA replacement, that is the existing CFC or HCFC needs to be removed and replaced with a single component HFA, for example HFA 134a or a blend of HFAs comprising HFA 134a and other HFAs. Thus, retrofitting HFA 134a into a system which was previously charged with a CFC or HCFC may require the system to be first flushed through with HFA 134a in order to completely remove traces of the original CFC and/or HCFC. This procedure results in a quantity of HFA 134a contaminated with the CFC and/or HCFC which, for both environmental and economic reasons, must be purified in order to remove the CFC and/or HCFC impurity which may then be safely destroyed, whilst the purified HFA 134a may be returned to the market.

Thus, for example, it may be desired, for refrigeration equipment which has previously employed dichlorodifluoromethane (CFC 12) as refrigerant, to replace the CFC 12 with HFA 134a. In such a case the equipment must be flushed through with HFA 134a in order to remove CFC 12 completely from the system. Such a procedure results in a mixture of HFA 134a and CFC 12 from which the CFC 12 must be removed before the HFA 134a is employed. This removal nay be effected by the process of this further aspect of the invention.

4

Mixtures which are treated by this further aspect of the invention typically comprise major proportions of hydrofluoroalkanes and minor proportions of the chlorine-containing fluorocarbon impurities, the actual proportions depending at least to some extent upon the origins of the mixture, the particular chlorine-containing fluorocarbon impurity to be removed and, where the mixture to be treated is a hydrofluoroalkane production process product stream, on the particular process employed.

Typically however, the chlorine-containing fluorocarbon impurity will have a concentrating in the mixture of at least 20ppm and even as high as at least 1000ppm. Indeed, the concentration may on occasion be much higher than 1000ppm. The mixtures may, depending upon the origin of the mixture, also contain unreacted reactants and other inorganic compounds which typically result from processes for the production of hydrofluoroalkanes, for example hydrogen fluoride and/or hydrogen chloride.

A particular advantage of the present invention for the removal of such impurities is that the impurities will usually be "in situ" converted to hydrofluoroalkanes. Thus, what may have previously been a highly toxic impurity may itself be converted to a hydrofluoroalkane which may be usefully employed with the hydrofluoroalkane already present in the mixture but which, in any case, is substantially less harmful than the chlorine-containing impurity from which it was produced by the process of the invention, and which may be removed easily from the resulting mixture by conventional techniques, for example distillation.

The invention is illustrated, but not limited, by the following examples in which all product yields are based on organic products only and were determined by Gas Chromatagram peak areas only. Solvent which had been carried over with the product was ignored when determining product yields.

EXAMPLE 1.

50ml of a 1.0M solution of $LiAlH_4$ in tetrahydrofuran (THF) were transferred to a 100ml glass round-bottomed flask. The flask was fitted with a Drikold condenser which gave sufficient cooling to prevent excessive carry over of THF but allowed the product to escape freely. The flask was maintained at $20^oC$ on an oil bath.

Chlorodifluoromethane was bubbled through the solution for 30 minutes at a flow rate of 10ml per minute. The products of the reaction were collected in a glass trap cooled in liquid nitrogen. The product mixture was analysed by gas chromatography and the results are shown in Table 1. No organic products other than those detailed in Table 1 were detected.

EXAMPLE 2.

The procedure described in example 1 was followed except that the $LiAlH_4$ in THF solution was maintained at $40^oC$. The results are shown in Table 1.

EXAMPLE 3.

The procedure described in example 1 was followed except that the $LiAlH_4$ in THF solution was maintained at $60^oC$. The results are shown in Table 1.

TABLE 1

| Temp. $^oC$. | %v/v difluoromethane | %v/v chlorofluoromethane | %v/v methane |
|---|---|---|---|
| 20 | 99.7 | 0.2 | 0.1 |
| 40 | 78.0 | 18.7 | 3.3 |
| 60 | 48.4 | 48.6 | 3.0 |

EXAMPLE 4.

6.3g of chlorodifluoromethane, 1.4g of $NaBH_4$ and 50 ml of iso-propanol were charged to a 125ml autoclave. The autoclave was heated, with stirring, at $70-75^oC$ for 4 hours. The gaseous products in the head space in the autoclave and the products dissolved in the solvent were analysed by Gas Chromatography and Mass Spectrometry. The only products were difluoromethane and unreacted chlorodifluoromethane. The proportions of these products are shown in Table 2.

TABLE 2

| | %v/v difluoromethane | %v/v chlorodifluoromethane |
|---|---|---|
| GAS | 67.1 | 32.9 |
| LIQUID | 22.3 | 77.7 |

## EXAMPLE 5.

The procedure of example 1 was followed except that 1,1,1,2-tetrafluorochloroethane was passed through the stirred LiAlH$_4$ solution at room temperature at a flow rate of 20ml/minute. The product vapour was sampled after 20 minutes, 40 minutes and 60 minutes and the samples were analysed by gas chromatography. The only products detected were 1,1,1,2-tetrafluoroethane and unreacted 1,1,1,2-tetrafluorochloroethane. The results are shown in Table 3.

TABLE 3

| Time/minutes | % v/v 1,1,1,2-tetrafluorochloroethane | % v/v 1,1,1,2-tetrafluoroethane. |
|---|---|---|
| 20 | 13.0 | 87.0 |
| 40 | 2.8 | 97.2 |
| 60 | 1.4 | 98.6 |

## EXAMPLE 6.

The procedure of example 5 was followed except that chloropentafluoroethane was passed through a 0.5M LiAlH$_4$ in diglyme (50ml) solution at ambient temperature at a flow rate of 20ml/minute. There was a 20°C to 30°C rise in temperature due to dissolution of chloropentafluoroethane/pentafluoroethane in the diglyme solution. After 80 minutes the reaction mixture had cooled to ambient temperature. The product vapour was sampled after 20 minutes, 40 minutes, 60 minutes, 80 minutes and 100 minutes. The samples were analysed by gas chromatography. The results are shown in Table 4.

TABLE 4

| Time/minutes | % v/v chloropentafluoroethane | % v/v pentafluoroethane |
|---|---|---|
| 20 | 55.3 | 44.7 |
| 40 | 39.8 | 60.2 |
| 60 | 53.2 | 46.8 |
| 80 | 62.1 | 37.9 |
| 100 | 69.2 | 30.8 |

## EXAMPLE 7.

The procedure of example 5 was followed except that difluorochloromethane was passed through a stirred solution (250cms$^3$ flask) comprising 100mls of 10% (w/w) LiAlH$_4$ in THF containing 9.6g of lithium hydride, at a flow rate of 40ml/minute. The temperature of the solution was maintained between 50°C and 80°C.

The product vapour was disengaged with nitrogen vapour (flow rate 330ml/minute) and sampled after increasing periods of time. The samples were analysed by gas chromatography. The results are shown in Table 5. The only products detected were difluoromethane and unreacted difluorochloromethane.

TABLE 5.

| Time/minutes. | Temp/°C. | % v/v difluoromethane | % v/v difluorochloromethane |
|---|---|---|---|
| 10 | 59 | 58.3 | 41.7 |
| 20 | 60 | 44.8 | 55.2 |
| 40 | 59 | 29.2 | 70.8 |
| 80 | 58 | 25.0 | 75.0 |
| 160 | 60 | 29.2 | 70.8 |
| 300 | 63 | 34.3 | 65.7 |
| 420 | 71 | 35.1 | 64.9 |

EXAMPLE 8.

The procedure of example 1 was followed except that difluoromethane doped with other fluorocarbons, and having the composition given in Table 6 was passed through a stirred 10% (w/w) solution of LiAlH$_4$ in THF at room temperature at a flow rate of 40ml/minute. The product vapour was sampled, downstream of an air-condensor used to minimise solvent carry-over, at various time intervals and the samples were analysed by gas chromatography. The results are shown in Table 6.

TABLE 6.

| Time | [Component] | | | | |
|---|---|---|---|---|---|
| | CH$_2$F$_2$ (%) | CH$_2$FCl ppm | CF$_2$Cl$_2$ ppm | CF$_3$CH$_3$ ppm | CF$_3$CF$_2$H ppm |
| 0 (Feed) | 99.52 | 1174 | 1075 | 1836 | 429 |
| 20 | 99.42 | 147 | 52 | 2400 | 319 |
| 40 | 99.53 | 287 | 62 | 1874 | 286 |
| 60 | 99.52 | 336 | 58 | 1816 | 286 |

Whilst the hydrofluoroalkanes present, difluoromethane, 1,1,1-trifluoroethane and pentafluoroethane were not affected by the hydride, (slight changes in concentration are due to selective solubility effects), the levels of the chlorine-containing fluorocarbons, dichlorodifluoromethane and chlorofluoromethane are substantially reduced. Advantageously, the dichlorodifluoromethane is converted to difluoromethane, the desired hydrofluoroalkane.

EXAMPLE 9.

The procedure of example 1 was followed except that 1,1,1,2-tetrafluoroethane doped with 1-chloro-2,2-difluoroethylene and dichlorodifluoromethane was passed through the stirred solution of LiAlH$_4$ at room temperature and at a flow rate of 50ml/minute. The product vapour was sampled, downstream of an air-condensor used to minimise solvent carry-over, at various time intervals and the samples were analysed by gas chromatography. The results are shown in Table 7.

TABLE 7

| Time (mins) | [Component] | |
|---|---|---|
| | CF$_2$Cl$_2$ ppm | CClHCF$_2$ ppm |
| 0 (Feed) | 2,200 | 546 |
| 24 | <300 | 138 |
| 53 | n/d | n/d |
| 80 | n/d | n/d |

**Claims**

1. A process for the production of a hydrofluorocarbon which process comprises contacting a fluorocarbon containing at least one atom of chlorine, bromine or iodine with a complex metal hydride.

2. A process as claimed in claim 1 wherein said fluorocarbon contains at least one atom of chlorine.

3. A process as claimed in claim 1 or claim 2 wherein said fluorocarbon contains at least two fluorine atoms for each carbon atom.

4. A process as claimed in any one of claims 1 to 3 wherein said fluorocarbon is passed through a solution of the complex metal hydride.

5. A process as claimed in any one of claims 1 to 4 wherein the temperature is in the range from about -80ºC to about 160ºC.

6. A process as claimed in any one of claims 1 to 5 wherein the complex metal hydride is a hydride of a complex of a group 1A metal and a group 3 metal.

7. A process as claimed in claim 6 wherein the complex metal hydride is lithium aluminium hydride.

8. A process as claimed in any one of claims 1 to 7 wherein a metal hydride is also present.

9. A process as claimed in claim 8 wherein said fluorocarbon is contacted with a solution of lithium aluminium hydride and lithium hydride.

10. A process as claimed in any one of claims 1 to 9 wherein said fluorocarbon containing at least one atom of chlorine is an impurity in a mixture thereof with a hydrofluoroalkane.

11. A process for the removal of fluorocarbons containing at least one atom of chlorine, bromine or iodine from mixtures thereof with hydrofluorocarbons which comprises contacting the mixture with a complex metal hydride.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | JOURNAL OF ORGANIC CHEMISTRY. vol. 32, no. 9, 12 September 1967, EASTON US pages 2749 - 2751; MARTIN H. KAUFMAN ET AL.: 'Synthesis of Terminal Perfluoromethylene Olefins' * page 2750, column 1, line 45 - page 2751, column 1, line 8 * * page 2751, column 1, line 25 - column 2, line 33 * | 1-2,5-7 | C07C17/00 C07C17/38 C07C19/08 |
| A | | 3-4,8-11 | |
| X | DE-B-1 062 695 (INTERNATIONAL POLAROID) * claim; example * | 1-2,5-7 | |
| A | | 3-4,8-11 | |
| X | DE-A-3 9D6 273 (GLAXO GROUP) * claims 1-5; examples 1-3 * | 1,5 | |
| A | | 2-4,6-11 | |
| A | EP-A-0 379 793 (ICI) * claims; examples * | 1-11 | |

TECHNICAL FIELDS SEARCHED (Int. Cl.5)

C07C

The present search report has been drawn up for all claims

| Place of search | Date of completion of the search | Examiner |
|---|---|---|
| THE HAGUE | 15 JULY 1992 | ZERVAS B. |